# EUROPEAN PATENT APPLICATION

(11) **EP 3 187 211 A1**
(43) Date of publication of application: **05.07.2017**
(21) Application number: 15203262.9
(22) Date of filing: 31.12.2015
(51) Int. Cl.: A61M 1/16, C25B 15/08, C25B 1/04

(54) **APPARATUS AND METHOD FOR GENERATING DIALYSATE FOR DIALYSIS**

(71) Applicant: Fraunhofer Gesellschaft zur Förderung der angewandten Forschung e.V., 80686 München (DE)
(72) Inventor: Goldau, Rainer, 23847 Kastorf (DE)
(74) Representative: Friese Goeden Patentanwälte PartGmbB

(57) **Abstract**

The invention relates to an apparatus and a method for generating dialysate for dialysis, the apparatus comprising an electrolysis unit (14) for electrolysing waste dialysate, a hydrogen separation filter (18) for separating hydrogen from the electrolysed dialysate, and a hydrogen fuel cell (21) for generating pure water with the hydrogen obtained from the hydrogen separation filter and oxygen from the enviroment.

## Description

The invention relates to an apparatus and a method for generating dialysate for dialysis.

### Background of the invention

The prior art apparatus and methods for generating dialysate for dialysis are facing the following problems:

### a) Availability of pure water

Availability of pure water is not omnipresent: Dialysate - the rinse fluid in close contact to the patients blood during dialysis - has to fulfill extremely high purity specifications. Complete absence of endotoxins, bacteria fragments and uremic toxins is essential to non-hazardous dialysis. Further specific concentrations of different types of ions, bicarbonate and glucose has to be matched prior to first contact to the blood.

A standard hemodialysis regime comprises 3 sessions per week, each 4h of duration with a continuous dialysate flow of at least 300ml/min. Therefore one week of dialysis of one patient requires approximately 2201 of ultrapure dialysate. The preparation of this amount is straightforward in countries where potable water and electricity is sufficiently available to operate reverse osmosis units from good quality tap water.

It is an insurmountable obstacle however in countries that are lacking these resources. Even the survival of patients who require dialysis is at risk under these circumstances.
It would be a great advantage if no pure water from external resources was needed for dialysis. The same applies to portable units having no access to external water resources.

### b) Removal of uremic toxins

Uremic toxins are manifold - as the methods adopted to clear them within one system:
- Activated char coal is used for adsorption of a diversity of molecule types
- immobilized urease is adopted to catalyze the urea - ammonia reaction with NH3 product as charcoal adsorption is low for urea and reverse osmosis cannot separate urea sufficiently alone.
- alternatively ammonium carbonate is generated and subsequently absorbed by Zirconium phosphate (REDY ® System 1)
- hydrated Zirconium oxide and carbon removes urea residuals, but does not remove the other uremic toxins.
- reverse osmosis filters remove further particles and ions if operated properly.
- many of the uremic toxins and retention solutes are still unknown or subject of open discussions.
- 56 of 88 solutes have been listed as uremic toxins later than 2007; 29 of them free water solutes - the process is still going on and it is uncertain what uremic toxins will be identified next.
- some of the smaller solutes cannot be separated by filtering or atmospheric pressure distillation as they are gas solutes or boil below 100°C (e.g. dimethylamine and other amines).
- some of them experience decomposition by heat (e.g. guanidine, proteins / middle-molecules), which cannot be controlled, because the size of compounds and type of removability (filtering, absorbing...) is unspecified.
- blood concentration limits for uremic toxins with respect to healthy individuals are still subject of scientific discussion. Therefore the skilled person does not have a valid specification of the reduction ratio to be accomplished. Accordingly it is difficult to choose an appropriate method.
- some constituents are deposited in quantities that are not compatible to a reverse osmosis filters function (urea and layer forming proteins).
- at high urea concentrations urea based chaotropic protein denaturation generates undefined protein decomposition successors. In technical terms there is a high variability of different substances with different approaches of removal. In particular reverse osmosis filters are prone to obstruction by these substances if their pores are of size below 1nm that permits urea removal.
- The more substances of unknown properties are to be extracted by a given process the more the technical approach of extraction will be a compromise.
- efficient reverse osmosis needs high pressures.

### c.) Power supply

In the vast majority of those countries where access to pure water is an issue also access to capable ac power supply is compromised. Electrical power is the most versatile form of energy which allows a high degree of technical freedom realizing different approaches to remove the uremic toxins. Driving pumps may be possible from accumulators, driving reverse osmosis units using high pressure will need mains access, driving distillation will need even more electrical power which has to be cooled and therefore is essentially lost. It would be advantageous if the dialysate purification device would be able to retrieve some of the energy invested or if a certain amount of purely thermal power can be utilized which is more omnipresent compared to electrical power (a simple combustion or the sun could deliver it). It would also be advantageous if the system was designed to work with low voltage as delivered by solar panels which become cheaper more and more.

### d) Non-stationary operation

Decentralized operation and in a second step wearability needs more than only independence from supply mains.

There are some essential and combined requirements that are advantageous to be fulfilled in order to allow decentralized (home dialysis) operation, overnight operation and even further to maintain wearability of an artificial kidney:
- The gross weight should be less than 6-8 kg (in a first approach, further reduction is advantageous).
- Outer shape should be designable such that it can be integrated into waist belts, backpacks or vests or at least is in a size able to be transported in small vehicles.
- Sufficient and equivalent clearance for all known and unknown uremic toxins, high dialysate purity.
- Independent of external power and water supply.
- Sufficiently silent to be worn or placed in meetings or quiet environments.
- No interference with orientation in gravity field (This might be problematic if distillation is used.).
- No disturbing or adverse exhaust, heat or malodor.
- Independent, maintenance free operation during reasonable periods like 4-8h.
- High safety requirements and safe shutdown or alarm in adverse situations, no regular daily medical supervision.
- Option of data logging and network connection to the hospital desirable to identify and remedy critical situations externally.
- Off facility simple and quick maintenance by the patient himself/herself without daily technical support and logistics.
- No replacement of disposables like sorbents, cartridges or substrates needed for filtering which cannot be accomplished by the patient himself/herself or which is not available at low cost.
- A reliable and sanitary vascular access that can be handled by the patient alone during all day dialysis.
- No cooling chains for catalytic agents.
- Absence of aluminum in absorbent / ion exchanger / urease carrier due to its compromised role in long term mental outcome and bone health.
- No additional sodium or pH buffer load by cations released in exchange to ammonium ions from ion exchanger / urease.
- Phyisiological dialysis would be a mandatory long term objective, e.g. controlling sodium, glucose and ultrafiltration according to internal body parameters, e.g. blood sodium or blood volume. A long term uncontrolled dialysis will have to closely follow the patients constitution and fluid state which may vary largely within the periods of new prescriptions by a physician.
- Finally the costs must be considerable low in particular in developing countries.

At the time being it might be acceptable that a few of these requirements are solved. They can only be an indication to not misdirect endeavor and focus on today's most important issues - which are still pure water generation, independence of power supply, high security of the device, and reliable blood access which together allow unsupported low cost home dialysis to the indigent at all - wearability in this sense is an advantage but not essential to survival of those who have no access to dialysis today.

### Prior art solutions

### a) Non-availability of water

Different approaches do exist. They all comprise the purification of ultrafiltrate from the patient within a feedback loop. Most prior art inventions comprise a closed loop circuit that conducts the ultrafiltrate drawn from the patient blood within a more or less regular dialysator to different kinds of units to purify them and afterwards returns it to the dialysate inlet. The water itself originates from the blood and stays within the liquid phase during the whole closed loop. Different approaches are used, even in combinations that cannot clearly be distinguished:
1. simple absorption of ultrafiltrate solutes or ion exchange and subsequent feedback (GB2124511, DE7315909, US2005274658).
   These Prior art documents comprise drawing ultrafiltrate from a dialyzer, conducting it on an adsorption unit or on a filter unit and feed it back to the dialysate. These solutions do not appreciate the inability of charcoal absorbers to adequately bind urea or the high pressures needed for hyperfiltration or reverse osmosis. Nevertheless they can be regarded as one of the early approaches to regenerate self drawn dialysate and realize wearable artificial kidneys. The circuit of hemodialyzer, ultrafiltrate regeneration and feedback is already disclosed. The use of activated charcoal is compromised due to its release of carbon fines17 which are suspicious to be pyrogen.
2. electrosorption (EP 2 087 916 A1)
   Materials with special affinity to uremic toxins are used as electrodes. Special materials with nanostructure, porosity or catalytic properties are used that bind uremic toxins. These methods have limited capacity and do not suit the need for high mass extraction.
3. nanofiltration, microfiltration combined with absorption or reverse osmosis (EP2277572B1,
   EP 2 281 591 A1)
   The dialysate is filtered by a nanofiltration unit (e.g. special materials or reverse osmosis) and subsequently routed upon an adhesive material to absorb those substances that still passed the nanofiltration.
4. electrocatalytic decomposition and electrosorption (EP 2 446 908 B1, EP 2 862 584 A1), in particular of urea.
   This approach comprises the decomposition and degassing of uremic toxins solved in the dialysate. Electrodes with catalytic or activated surfaces are used and supplied with specific electric current. Also electronic means are taken to avoid electrode degeneration. The materials are mostly from the group of nanostructured sorption material, polymeric matrices or specific metals like Pt, Ni, Ti, Ir, Sn, Ta or Ru, oxides, oxyhydroxides and Hydroxides and are shaped plane, circular, tubular or like filaments/threads. In particular urea can be decomposed by electrolysis at a much lower voltage than needed for water electrolysis. Also cartridges are suggested to ease handling. This principle does not work for all solutes and cartridge logistics can be an issue.
5. graft on demand by bioartificial tissue engineering (partially quoted from US 2015/0093812 A1)
   A decellularized seeding system is used to seed glomeruli-type structures on a filtration organ scaffold (lung or other organs like kidney), which are cultivated under controlled pressure environment. Even afferent and efferent vessels are generated by this technique. After a seeding phase the initial kidney constructs can be transferred to a perfusion bioreactor to provide whole organ culture conditions and bring the tissue to the next step of maturity. Finally it can be used for filtration - described here very briefly. This approach seems to be very promising in the long run but today is still far from being applicable to humans.
6. electrochemical detoxification (US3 878 564)
   Using electrolysis most of the solvents are regarded to be oxidizable by radicals generated by electrolysis or are converted into harmless products like water, CO2 and nitrogene. Some of the files lack of detailed chemical reaction disclosure, but for urea it works and can avoid sorbents. Also an electrochemical ammonia reactor is comprised. Substances listed here comprise NH, NH+, urea, uric acid, digitalis glycosides, carbon monoxide, barbiturates, ketone bodies, aceto acetate, methanol, creatinine, alanine, ethanol, and mixtures thereof. The drawback is that radicals and chlorine oxidants may occur, any of them must be safely reduced by additional charcoal layers before the water may have blood contact again. There is a certain hazard risk.
7. urease based decomposition and ion exchange layer in sorbents (e.g. US 4 581 141, similar Redy ® system and later derivates like Allient® or Dialysorb®)
   This is the most promising approach. Urease is immobilized (sometimes on an aluminum containing carrier) to act on urea which decomposes to bicarbonate and ammonium. The system is integrated into one of the known hemodialysis methods. The basic principle after urea decomposition is ion exchange and catalytic conversion. The large amount of ammonium to be exchanged before dialysate may have contact to blood enforces high amounts of ion exchange mass. The protons generated from urea by urease tend to decrease pH and therefore are a load to the bicarbonate buffer. The system therefore is discussed to be suitable for bicarbonate dialysis.
   There is a risk of a filter break through when overloaded with ammonium. Further the ions exchanged may adversely influence the body's electrolyte balance and pH buffering. Aluminum carriers have been discussed to be very critical.
   This principle does not cover all substances but addresses one of the most important waste dialysate constituents - urea - properly and has undergone essential development and a certain acceptance but is still not widespread accepted due to costs, logistics, some longterm outcome concerns and the filter breakthrough/overfeeding apprehension mentioned.
8. combination of ion separation with electrodialysis and enzymatic urea decomposition (DE 3010414 A1)
   Electrodialysis is a principle which comprises several chambers, each with two electrodes of different polarity. The chambers are separated by the electrodes themselves and the electrodes are semipermeable for the ions they do not attract by their polarity. Therefore different chambers can be located contiguously and build a system of high throughput which is capable to reduce ions.
   Different types of membranes and carrier molecules are used to address specific ions. Not all of the substances can be addressed.
9. other combinations of the above mentioned systems are envisaged (WO95/02559)
   In this prior art document an electrolysis cell is used to generate hypochlorite, the fluid is then conducted to a degassing unit, a charcoal filter unit and a zeolite filter unit using urease. The detailed reasons for the combination has not been disclosed.
10. electrophoresis blood purification (US2003187380A1)
   An electric field is applied to remove metabolic components from plasma or blood using membrane based electrophoresis. Applied voltages are up to 200V and even hemolysis is discussed (0.05% of red blood cells lysed after single pass). Many different types of substances can be addressed, but the mechanism of specificity is not laid open within this prior art document.
11. An approach to clear dialysate using distillation of water has only been found for spacecraft systems (US20140061127).
   Distillation is a principle that addresses the water and low boiling solutes but can only work in continuous flow if no low-boiling solutes are existing. Many of the amines found in urine are low boiling and therefore may be transferred to the condensate. Further gravity aspects need to be taken into account. Distilling water in all positions may impose some technical issues and requires high energy consumption.

Also a bioreactor to contact urea to immobilized urease (granulated activated carbon-urease) is mentioned this prior art document, with the generated ammonia conducted to a subsequent electrochemical cell to provide electrical energy. Finally molecular nitrogen and water is produced. This system aims to not loose any of the substances. Forward osmosis that in a first step extracts water from the system by osmolarity is the only principle aside distilling that address the solvent and not the solutes. This is advantageous, but if osmotic forces are used high osmolarity is needed. Although the engineers are free to use ions that suit the needs a further reverse osmosis step is needed to extract the water again from the high osmotic solution. It reduces the number different solutes offered to reverse osmosis but is water consuming. Further it cannot separate urea that comes with the solvent drag.

Also UV irradiation has been suggested, which does not extract solvents and therefore is not a convenient solution for generation of ultrapure dialysate.

The above listing of the different solutions to purify dialysate from uremic toxins demonstrates that there are very specific solutions to specific solutes and a manifoldness of different approaches.

The solutions to clear the ultrafiltrate are diverse and use many different techniques including effects of an electric field on the solutes, adsorbing the solutes, subject them to chemical reactions mainly of catalytic type or immobilizing them on specific membranes. A technical system dealing with all relevant - and still unknown - substances will surely consist of combinations of the above mentioned methods. This makes the devices complicated.

Accordingly it is an object of the invention to provide an apparatus and a method for generating dialysate for dialysis which does not require pure water for purifying dialysate.

### b) Uremic toxins

In the prior art there is diversity of methods to remove uremic toxins from the dialysate.

Adsorption, catalytic reactions and ultrafiltration / reverse /forward osmosis are used for urea and middle size molecules:
Urea may be regarded to be the lead substance of uremia - as the name indicates- thus urea is a main constituent of the human urine. Urea cannot be adsorbed in high amounts by charcoal. 200g of active charcoal are capable to absorb 2-3mg of urea. Urea excretion is estimated to be 5.7 mmol/d/kg of bodyweight. This is for an 75kg person 430mmol/d or 25g/d, strongly depending on the diet. This amount cannot be absorbed by charcoal in reasonable quantities. Therefore reverse osmosis (RO) is considered to remedy the situation. RO is not the favourite method to remove urea, as urea molecule radius is in the range of 0.18nm. This is quite similar to water (which has a diameter of 0.31nm or a bit less calculated from molar volume). Therefore urea as an undissociated molecule, which experiences no charge repelling forces, can hardly be extracted just by pore size and/or a molecular sieve.

Further pore sizes and/or a molecular sieves are even a bit larger by technical reasons, as the overall fluid must be sufficient and the pressures must not exceed the membrane stability.

The effective membrane pores (there are not really pores, it is just a model) show calculatory equivalent sizes of 0.33nm to 0.44nm8. Typical rejection rates are as low 0.2-0.3 therefore.

This means 70-80% of urea are still found in filtrate after single pass RO. Several equivalent passes (recirculations) with additional pump energy input are needed. Further the filter water is also split per RO pass. Typically only 50% pass the membrane. If 70% of urea pass the membrane, but only 50-60% of the water, urea is enriched in the filtrate - contrary to all the other solutes that are retained. This is due to the water ability to build conglomerates. This makes RO infeasible if applied also for non urea solutes.

Most ultra low pressure membranes are multi-layer thin film composites of polymers. The transport process therefore has diffusive character obeying Stoke's equation and is prone to obstruction by the load. The retention rates for urea are 0,17mmol/l. Typical plasma urea concentrations which would roughly appear in dialysate would be 5mmol/l, which is 30 times higher. It is unclear whether filters will work sufficiently for prolonged periods at higher concentrations. Even NASA related scientists Cabrera et al. are led "to the conclusion that urea is still hard to reject by such membranes" (US20140061127).

Further high effort is taken to make RO units work reliable and enduring. Substance layers stemming from filtration solutes are obstructing the membranes and its interior reducing effectivity or increasing fouling. In particular rotational movement of the membrane (which is additional mechanical effort) seems to have counter-fouling effect and is discussed for spacecraft water reclamation.

If the reverse osmosis system is used in the immediate vicinity of the patients there is a further serious drawback:
Any reverse osmosis system needs pumps that generate pressures in the range of 500kPa-4000kPa or even more and therefore will not work completely silent. It is a challenge of mechanical construction to make them operate at sufficiently low noise in quiet environments as necessary if a patient uses the device in her daily life.

Urea decomposition by enzymes (urease) is a method to make urea fragments accessible to further treatment like ammonium electrolysis cells or separation. NH3 is a high energy molecule which can be metabolized to molecular nitrogen and water.

Other small and middle-molecule solutes can more easily be filtered by size exclusion even if the rejection rates are far below and not optimal. Reverse osmosis filters are established to clear any substances of significantly higher molecular radius than water as long as the load is not too high in absolute mass. RO systems will cover all specifications related to purity here - they will not cover the specifications with respect to water savings - as the retentate is lost - and to noiselessness. Energy supply for the pump must be electrical and is in the range of 10W per 1/h.

Methods like adhesion, adsorption, reverse and forward osmosis, catalysis or other chemical reactions and distillation are quiet elaborated meanwhile but may still suffer from inconvenient durability, reliability, specificity and functionality for a patient near or patient-worn system. It is not easy to build a technical system that fulfils the demands for removal of many different substances, part of them unknown. It would be advantageous to concentrate on only a few or only one substance. Further the costs of applying these methods (e.g. sorbent cartridges) are too high for those living in countries where water is scarce.

Accordingly it is another object of the invention to provide an apparatus and a method for generating dialysate for dialysis which is essentially independent from the uremic toxins in the dialysate.

### c.) power supply issues

Any of the prior art dialysis systems need their energy in electrical form, most of them using AC. In particular wearable artificial kidneys discharge accumulators when being worn and need at least temporary mains supply to recharge them - which might be acceptable during periods of the patient being at rest. The drawback is that there are many countries with enormous numbers of end stage renal disease (ESRD) patients that cannot maintain a stable and powerful decentralized electrical network, besides the water lacking. Even for hospitals this can be a challenge, in particular in crisis situations - dialysis cannot be interrupted for weeks during hospital recovery after war impact. A conventional hemodialysis device will need at least 1000W of reliable, uninterrupted AC electrical power during certain periods of rinsing, cleaning or heating and a few hundred W during regular treatment. It would be desirable to perform dialysis independent from external mains at lower voltages as these can more easily be supplied by solar panels in many of those countries lacking of reliable power network.

When developing a home dialysis device or even a wearable artificial kidney in a first approach, it should be acceptable to use electrical power temporarily even from cord. However a further development step should be the complete independence from high current electrical supply. A solar panel might be a reasonable compromise to operate or at least to start the artificial kidney operation until internal processes omit the need for external supply for an appropriate period. Thermal energy as primary source would be advantageous due to its much higher availability compared to electrical power. It would be even more advantageous to use additional means of power storage in the form of chemical energy.

Accordingly it is another object of the invention to provide an apparatus and a method for generating dialysate for dialysis which is energy efficient.

### d) Decentralized operation as an artificial kidney

The outstanding issue beside power supply is the lack of water availability. Water is not omnipresent in countries of equatorial latitudes, but solar power might be sufficient - in particular if there is a way to store energy in chemical form. All other shortcomings like logistics for maintenance, filters and other spare-parts like cartridges or lack of local technical and medical expertise in principle can be resolved even in less developed countries compared to the dialysis water deficiency, which is unresolvable.
To address this problem the prominent solution suggested in the prior art patents is the use of reclaimed dialysate or ultrafiltrate from the patient himself/herself within a feedback loop and reclaiming it using one or several of the methods mentioned above. This principle has been suggested in numerous prior art documents (US2003097086, EP2281591,US2010100027, WO09083011, WO11031610, DE7315909) and has already led to wearable artificial kidneys.

Probably not all requirements decentralized or wearable dialysis imposes could be fulfilled in a first approach as the past has shown. Although different and encouraging devices during the last 35 years have come up and demonstrated that wearable artificial kidneys are possible and can keep patients alive for periods measured in years, these systems have not found widespread acceptance up to now. Not only technical restrictions but also some concern about the treatment equivalence, toxicity and filter break through, related with drawbacks to electrolyte and acidity metabolism, home dialysis vascular access handling and long term outcome may be the reason, as well as the costs for adsorbent cartridges. One may speculate if there are other reasons than technical inconvenience - but doubtlessly still technical aspects are not satisfying. Particular effort is necessary to address essential requirements like power and water independence, uncompromised clearance equivalence, silence, handiness and in case of intended wearability low weight. Further a very silent internal power generation would be advantageous to at least keep the essential components like pumps, control logic and external connections operating if accumulator capacity is running short.

Systems that address the need of indigent patients that cannot pay for continuous medical supervision are further obliged to be very moderate in treatment costs and the treatment handling of such systems without continuous medical support must be simple, inherently safe and easy to handle. To imagine a trained patient performs dialysis by himself/herself for longer periods (several months) without medical supervision (as an alternative to death) implies a much more crucial clearance and blood parameter observation than in hospital dialysis. The system should further be able to clear and purify reuse filters with pure water.
One of the remaining drawbacks of all existing technology that works with ultrafiltrate is that it can only act adequately on known solutes. Unknown solutes may or may not be effectively cleared from the dialysate and have potential to enforce technical modifications. Beside a non-total solute removal of the known uremic toxins this is the reason why it cannot be guaranteed that the treatment quality is equivalent to a pure water and reverse osmosis fed hemodialysis system. It is obvious that matching all requirements is still an enormous endeavor and it would also be a significant leap to address the majority of them successfully. The present invention is aimed to improve some of them, not all. In some aspects it goes a bit further than existing techniques.

Methods like distillation, adsorption or using high pressure pumps (reverse osmosis) do not fully match essential requirements like sufficient independence of electrical energy or water supply, handiness, treatment equivalence and costs. Toxicity from cartridge released substances and infections are further concerns. There are still unresolved issues.

Accordingly it is another object of the invention to provide an apparatus and a method for generating dialysate for dialysis which is independent from external water and energy supply.

### Summary of the invention

One or more of the above mentioned objects of the invention are solved by the apparatus and method as featured in the independent claims. Preferred embodiments of the invention are specified in the dependent claims.

According to the invention there is provided an apparatus for generating dialysate for dialysis comprising an electrolysis unit for electrolysing waste dialysate, a hydrogen separation filter for separating hydrogen from the electrolysed dialysate, and a hydrogen fuel cell for genarating pure water with the hydrogen obtained from the hydrogen separation filter and oxygen from the enviroment.

According to the invention the electrolysis unit can be a steam electrolysis unit.

According to the invention the electrolysis unit can be a solid electrolyte cell operating with solid oxide electrochemical conversion.

According to the invention the electrolysis unit can be operated with alternating polarities of the electrical power.

According to the invention the electrolysis unit can be a low temperature electrolysis unit.

According to the invention the anode and cathode electron transfer processes can be spatially separated with the goal of cell voltage reduction and where additional energy is won from chemical bonds of energy carriers or is intermediately stored in additional catalysts, additional intermediates or molecules that can be spatially transferred and which are converted within a redox cycle with the same net electron transfer result as in electrolysis. An example would be the Copper-Chlorine-Cycle or the use of carbon electrodes.

According to the invention the hydrogen separation filter can be is a pore size filter and/or a molecular sieve.

According to the invention the hydrogen separation filter can be a microporeaus ceramic compound.

According to the invention the apparatus can be separated into a base unit and a portable unit.

According to the invention the apparatus can comprise a docking interface for connecting the base station with the portable unit.

According to the invention the apparatus can comprise a pluratliy of docking interfaces for connecting a pluratilty of protable units with the base station.

According to the invention the docking interface can comprise interfaces for connecting reservoirs provided in the base station and the portable unit.

According to the invention there is also provided a method for generating dialysate for dialysis using an apparatus of the invention as mentioned above.

According to the invention there is also provided a method for generating dialysate for dialysis with the follwing steps: electrolysing waste dialysate using electrolysing unit, separating hydrogen from the electrolysed dialysate using a hydrogen separation filter, and genarating pure water with the hydrogen obtained from the hydrogen separation filter and oxygen from the enviroment with a hydrogen fuel cell.

According to the invention the optimum working temperature of the hydrogen sepration filter can be higher than 130°C, preferably higher than 150°C, more preferred higher than 180°C and most preferred about 200°C.

According to the invention the optimum working temperature of the hydrogen sepration filter can be less than 480°C, preferably less than 350°C, more preferred less than 250°C and most preferred about 200°C.

According to the invention the optimum working temperature of the electrolysis unit can be at about the same temperature as the optimum working temperature of the hydrogen sepration filter.

According to the invention the electrolysing step can comprise steam electrolysing.

According to the invention the electrolysing step can be operated with alternating polarities of the electrical power.

According to the invention the electrolysing step can use a low temperature electrolysis unit.

According to the invention the electrolysing step can comprise spatially separting the anode and cathode electron transfer processes with the goal of cell voltage reduction and where additional energy is won from chemical bonds of energy carriers or is intermediately stored in additional catalysts, additional intermediates or molecules that can be spatially transferred and which are converted within a redox cycle with the same net electron transfer result as in electrolysis. An example would be the Copper-Chlorine-Cycle or the use of carbon electrodes.

According to the invention the hydrogen separating step can use a pore size filter and/or a molecular sieve.

According to the invention the hydrogen separating step can use a microporeaus ceramic compound.

One basic idea of the invention is to extract the water from the ultrafiltrate drawn from a conventional dialysis filter using different kinds of electrolysis. Thereby it is not necessary to extract most or all of the uremic toxins from the ultrafiltrate because the ultrafiltrate will not be needed in contrast to the prior art. This makes it possible to concentrate on only one technical solution which is free from any adsorbents or ion exchangers or disposables. The hydrogen generated by electrolysis will be conducted through a gas separation membrane that is only permeable for hydrogen - due to its small size it can be separated from any gas mixture. Afterwards the pure hydrogen can be fueled to a fuel cell generating water with the environmental oxygen (O₂).

This water is preferably provided with additional electrolytes, bicarbonate and other necessary substances and then fed back to the dialyzer.

On the first glance the skilled person would not consider this solution of the invention because of the relative high energy needed for the electrolysis. However the energy needed for electrolysis can be regained in parts from operating the fuel cell for generating pure water with the environmental oxygen. The energy investment for the electrolysis essentially has to replace only efficiency losses and therefore is depending on only the technical development of the future which is moving forward quickly as hydrogen is the energy of the future to help solving climate crisis. If both steps have 80% efficiency, 64% of the energy invested can be regained. The energy in parts is electric and in other parts thermal or chemical energy. Using solar panels for both makes the principle feasible for developing countries where solar energy usually is abundant. For an elaborated system the electrical energy needed for dialyzing one patient with continuous low flux dialysis has been calculated to be in the range of 1-3 kW, which can be delivered by solar panels easily in equatorial latitudes. Solar panels in particular match the electric requirements of electrolysis cells (DC, low voltage).

The apparatus and method in accordance with the invention has the further advantage that it is independent from the nature of the uremic toxins in the blood of the patient. Therefore in contrast to the prior art solution the invention does not need to have any information about substances being uremic toxins, because all substances except water are removed. In the prior art the advent of the knowledge that a new substance is a uremic toxin may have implications on the technique used for its removal. As this occurs frequently it is advantageous to concentrate on only one substance that will never change and is essential to dialysis. According to the present invention this substance is water. This allows to confine to only one technical principle at all which can be highly elaborated as it is no technical compromise.

Water could also be extracted by few-stage distillation if there weren't additional low boiling substances like some amines that might be transferred to the condensate. Even though this could be handled by specific temperature steps during temperature rise or by under-pressure, the energy invested for distillation is finally lost and must be cooled. The loss of energy can be avoided if distillation and electrolysis are combined or if electrolysis is used alone (cold electrolysis).

Due to energy recovery it is energetically more efficient to use electrolysis than distillation - and not to extract the uremic toxins but to extract the water only from the ultrafiltrate. The electrolysis result will essentially be hydrogen gas H₂ (cathode), chlorine gas Cl₂ (depending on the amount of NaCl in the electrolyte and type of the diaphragm), nitrogen and carbon dioxide from electrolytic urea decomposition and in absence of Cl⁻ and some further impurities also oxygen gas O₂ (anode) originating from the water. If electrolysis is performed at low temperature (<100°C) within spent dialysate H₂O(liquid) the presence of Cl⁻ from sodium chloride and the subsequent generation of hypochlorite and chlorine is disturbing but not preventing the principle from operation: According to the invention a membrane for gas separation is used to have only access to filtered H₂ from the gas mixture. It is essential to have the hydrogen generated by electrolysis subsequently passing a size selective filter membrane that makes use of H₂ being the smallest molecule of all. The diaphragm can be omitted when an effective H₂ filter exists for gas separation. Using such a filter membrane in principle permits to omit the electrolysis semipermeable membrane at all that prevents the gases from mixing - as H₂ separation from all the other gases is performed afterwards and other technical means are taken to avoid explosion.

In case of using nickel electrodes also urea is electrolyzed first due to its lower standard potential and will generate N₂, CO₂ at the anode and H₂ at the cathode. It is not necessary to wait until no more chlorine, nitrogen and carbon dioxide are generated at the anode before the O₂ generation sets on - because O₂ is not needed, the fuel cell can finally aspire it from the surrounding atmosphere. Therefore the dialysate is already pre-purified - but not sufficient to be already used as reclaimed dialysate. The low temperature electrolysis will have to be continued until urea and Cl⁻ and finally all water is electrolyzed. The H₂ generated is filtered and afterwards combusted to pure water in a fuel cell with environmental O₂. Nevertheless it can be helpful to first perform a low temperature urea electrolysis to get rid of the urea in gaseous forms as urea is the main contributor to process deterioration and waste volume. Electrolysing also urea may prolong the maintenance interval it takes until the device must be cleaned by the patient.

According to a preferred embodiment steam electrolysis is used. Considering the overall energy needed for heating the water and electrolyzing the water steam, steam electrolysis needs less energy than cold analysis due to the lower energy needed for the electrolysis of water steam compared to the electrolysis of liquid water.

It is thermodynamically even more efficient to use higher temperature steam electrolysis (at approximately 200°C) (even but not exclusively below urea decomposition temperature of 480°C), which implicitly uses distillation to generate the steam. It shifts -depending from temperature and current - the anode potential from 1.23V to the region of 1V in the gas phase (hot steam) - thus decreasing the electrical part of the energy and increasing the thermal part. Also gas bubbles in fluids are avoided using steam - one of the major disadvantages of fluid electrolysis cells that is partially limiting the operation current. So steam electrolysis further prevents Cl⁻ or urine from being present at the anode and therefore prevents chlorine, carbon dioxide and nitrogen from anodic generation. By steam electrolysis part of the entropy gain can be invested by cheap thermal energy (chemical combustion) instead of expensive electric energy. The H₂ from the cathode can be extracted from any gas mixture using modern ceramic compound membranes due to its small molecular diameter of only 74pm (compare water: 310pm without hydration cluster). Using moderate high temperature electrolysis (∼200°C or even more) would also allow to use solid electrolyte cells (SOEC, solid oxide electrochemical conversion) and is hygienic per se. But even the use of electrolytes (solid oxid electrolyte or fluid electrolyte) at all is not really necessary if the cathode and anode gas must not be separated as the migration of the ions can be avoided using AC or AC pulses at convenient frequency. The subsequent filtering will assure that H₂ finally is separated from Cl₂, O₂ and others under any circumstances and the gas separating element of the electrolysis cell is not really needed.

Depending on the type of electrolysis chosen the increase of pressure within the electrolysis cell can also improve energy efficiency to a certain extent. It should be considered in cases where the generation of already compressed hydrogen is helpful, e.g. for storage of over-day generated hydrogen to perform overnight dialysis from it.

As means to further increase electrolysis efficiency also AC drive or pulsed DC drive cell power supply as well as reduction of the electrode spacing of the electrolysis cell can be considered. This will reduce dissipative Ohmic losses at the cost of higher impedance of the cell.

Further changing polarity may enhance electrode lifetime and maintenance intervals.

In some cases it might be necessary to use carrier gases (argon, nitrogen) when performing steam electrolysis. The carrier gases can be reclaimed when using a H₂ separation membrane and therefore is not lost. Once H₂ is separated at high purity by a ceramic compound membrane or a functionally similar type it can be combusted in a fuel cell using environmental oxygen. This avoids to use the retentate gas from the filter membrane with its questionable impurities and generates nearly the same amount of water as invested. The water from the fuel cell is of high purity, free of any uremic toxins and can be used for dialysis after being prepared with electrolytes and buffer to match metabolic requirements for feedback to blood contact within the dialyzer. The electricity from the fuel cell can be used to partially drive the electrolysis and therefore reclaim parts of the energy invested. Only the dissipation energy of the whole circuit must be reinvested, depending on the total effectivity of the two cell types which can reach 80%. Some advantageous embodiments of the dialysis system include the time separation of H₂ generation and its fueling to the fuel cell and subsequent use for dialysis. H2 in this case could be intermediately be stored in steel gas cylinders or other H2 storage units. This allows to produce it during daytime from solar panels, which produce low voltage power very convenient for electrolysis cells - and to dialyze the patient during night time from the stored H2. A 300 hPa 201 cylinder can store enough H₂ for an 8h low flux dialysis if a fuel cell is used during the night to power the electrolysis cell also during night. This makes the complete system suitable for areas with high sun irradiation, low water resources, and indigent patients who are not able to pay additionally for adsorber cartridges.

According to the invention waste ultrafiltrate (which otherwise would be discarded) can be electrolysis/fuel cell purified at the choice of the patient and not be used for dialysate but for cleansing purposes. The patient who intends to reuse disposables due to his situation can use this pure water for rinsing - although reuse might be a compromise it is a fact to save money.

The purified water from the electrolysis and fuel cell can also be primed with some sodium chloride added after purification and again be electrolyzed at low temperature in liquid form in a separate cell.

According to the standard- and over- potential chlorine will be formed within the water to react with the hydroxide ions from H₂ generation to hypochlorous acid. This is called electrolyzed water and can be used as disinfectant for different purposes, e.g. cleaning the device.

The water gained from ultrafiltration and purified by an electrolysis - fuel cell combination can be used also for preparation of peritoneal dialysis dialysate as it has sufficient purity.

According to the invention the amount of electrical energy needed for the electrolysis can be reduced further using metal redox cycles. Some of these few hundred cycle types known are at sufficiently low temperature to be handled by a hospital based station. A preferable but not the only type is the Cu-Cl cycle which works at a temperature maximum of 530°C. It needs significant additional machinery that cannot be handled by the patient alone. This approach is particularly useful as a dialysate base station for hospitals with compromised power and water supply but reliable access to solar or carbon fueled heat. Using a metal redox cycle the dialysate preparation for several patients at a time can be considered even in combination with the intermediate storage of H2 in gas cylinders aforementioned for later use and therefore decoupling dialysis and solar irradiation. The Cu-Cl cycle has less electrical power consumption but higher thermal energy consumption than regular electrolysis and therefore shifts the demand more to the given situation found in equatorial latitudes where solar heat and only solar electricity from panels is available.
High temperature steam electrolysis in combination with solid oxide electrolytic cell is another option to shift the energy invested to a higher thermal fraction. In this case effectivities up to 59% (solar irradiation versus H2 output energy) in ideal situations are reported, which is nearly thrice the value of photovoltaic solar panels. As only steam is used this still works with impurities in the feedback dialysate. Although this approach due to its high temperatures is demanding to the vessels and housings on the other hand the focusing of solar heat to generate steam and to install a solar panel for electricity generation would only demand 8 m2 of perpendicularly irradiated surface in countries of the equatorial region, only part of it as a photovoltaic panel. This would allow to generate pure H2 during the day sufficient for one patient and generate H2O for dialysis during the night.

As in developing countries power shortage may be frequent it is helpful to further shift to fewer electrical power requirements that can easily be satisfied by solar panels alone. Again this is associated with additional machinery and therefore only suitable for hospital base stations that serve for several patients. A convenient method is to use carbon slurry or carbon electrodes and a H₂SO₄ electrolyte to generate H₂. The anode standard potential can be reduced to 0.21V using coal slurry. In this case the reduction of the standard potential of water electrolysis (1,23V) to only 17% directly scales to the theoretical/practical saving of 83%/52% electrical energy compared to regular low temperature water electrolysis. The energy is still needed but no longer is contributed thermally but chemically by providing the coal slurry which can easily be milled from coal and locally be prepared. Coal in many countries is one of the cheapest energy resources as it can also be wooden coal. The coal -water mixture is decomposed to CO₂ and H₂ by electrolysis. H₂ again can be separated by size sensitive membranes and afterwards be fueled to a fuel cell to form water with oxygen from the environment. Therefore the water generated is very pure and not compromised whatever chemistry has happened before. The water in the H₂SO₄ electrolyte lost by electrolysis has to be replaced by ultrafiltrate originating from the patients. Also the coal has to be replaced if an aliquot of the energy needed is drawn from the carbon of the coal. At the cost of an additional coal fueling this electrolysis cell is able to produce 2H₂ for every single C atom contained in the coal. The coal slurry standard potential is lower than that for the Cl-or urea oxidation and therefor is not competing for the current delivered, e.g. from a solar panel. To generate 1,31/h of pure dialysate (which is the non-stop equivalent of a 3x4h weekly dialysis with 300ml/min dialysate flow) approximately 20m² of solar panel (η=0.16) would be needed for one patient using this method. From time to time it can be helpful for decreasing electrode potential to also oxidize the other constituents mentioned above originating from dialysis. But the uremic toxin waste can also be discarded with the inactivated coal slurry finally as this happens frequently.

Urea can also be split by electrolysis at a standard potential of 0.37V (compared to 1.23V of water) and therefore would occur during low temperature electrolysis in the spent dialysate if water electrolysis is performed.

The H₂ filter may consist of different materials with different effectivity. The filter types available and convenient for given temperature range, separation quality, flow rate and poisoning agents can be found in a selection table accompanied by a listing of the major properties of the different membrane types. For the most interesting temperature region around 200°C a microporous ceramic compound type working as a molecular sieve may be advantageous - even though there is still some room for technical improvements. These ceramic compounds have potential to be produced in higher numbers and can be expected to fulfill the demands of purification of H₂ for dialysis in sufficient amounts.

As high quality preparation of dialysate from virtually any water source is possible with such a device it can also be considered to obtain water from animals or other sources using old dialysis filters that can no longer be used for men due to their pore properties and operation time elapsed. This would allow to employ the kidneys of animals and their water resources for dialysis - if they are able to stand the immunological irritation such a procedure induces. In such a circuit the spent dialysate would directly be infused into animals like cows, camels or pigs with sufficient robustness and body volume to have their kidneys purify the waste dialysate. At other location the blood is drawn from the animals vein and conducted to an old, reused dialysis filter to separate their extracellular blood water. The animals ultrafiltrate from this filter - bearing only uremic toxin concentration and protein content of healthy animals - is electrolyzed according to one of the principles mentioned and afterwards the H₂ is pressed through a molecular sieve membrane and be fueled to a fuel cell to generate water of high purity. This water - after adding of electrolytes and others - can be used for dialysis. The dialysis can be performed at a later time as the extraction of the fluid from the animal. The advantage is a strong reduction of uremic toxins collected in the device and therefore longer maintenance intervals as also the residuals of the water electrolysis can be rinsed back to the animal. The cleaning and disinfection of the device therefore could be reduced. Also under-hydrated patients could be dialyzed this way if the initial ultrafiltration of water to operate the device internal circuits is impossible. And animals are an additional water source in areas where no other water is available due to harsh drought - as they sometimes are able to extract water from sources inaccessible to humans. Finally the ability to convert animal water could help spreading such dialysis systems and its costs as they may be misused for potable water generation if not performing dialysis.

The waste from the electrolysis cell will have to be discarded frequently. This enforces the construction of it in a way that the patient can discard the residuals and clean the electrodes without technical assistance. One embodiment of this could be the use of cartridges. Another embodiment is to construct the electrolysis cell in a way that it can be cleaned by the patient himself/herself using water or disinfectant generated by the device itself.

For the generation of 1.3kg water/h an electrode surface of approximately 2-3 m² is needed. By packing the electrodes in folded and serialized compartments and reducing their cross section and thickness to the minimum needed to bear the current even this surface is compatible to wearability.

According to the invention the components of the apparatus can be separated in stationary and portable components to enhance its wearability.

According to the invention the apparatus can be separated into a basis unit (or basis station) and one or more portable units. In case of more portable units these portable units may be used simultaneously, if the basis unit has an appropriate size.

The basis unit may comprise a main reservoir for used dialysate, a electrolysis unit, a hydrogen filtering unit, a hydrogen fuel cell, a main reservoir for fresh water generated by the fuel cell, a main hydrogen reservoir and a pump for pressurizing the gaseous hydrogen, a main store unit for additives (electrolyte, buffer, anticoagulates, etc.) to be added to the water to obtain fresh dialysate, a pump and control unit, interface means for charging and discharging the portable unit within a short time (e.g. 2 min).

The portable unit may comprise a small reservoir for fresh and used dialysate, a pump device for pumping blood and dialysate, a dialysis filtering unit to be operated in countercurrent, a dosage unit for adding additives to the fresh water to obtain fresh dialysate, a control unit, a small hydrogen reservoir and a hydrogen fuel cell (for running the protable unit for a few hours), a small accumulator to be used for starting up the protable unit when the fuel cell is not running, and an interface machting with the interface of the basis unit, such that dialysate can be discharged and water, electrolyte, additives, hydrogen and be refilled for running the portable unit and its fuel cell. The components should be designed to meet the needs of a portable device, i.e. the total weight including materials should not exceed 6 to 8 kg.

Alternatively and/or in addition to the small fuel cell the portable unit may comprise a hydrogen burner (flame or catalytic) wherein the complete dialysate can be obtained from the reaction of hydrogen and oxygen in the detonation gas reaction. The portable unit will get the hydrogen needed from the base unit to be operated for a few hours. About 500g H₂ should be sufficient for operating the portable unit for a few hours. 500g H₂ should result into 4.5 kg water if the oxygene is obtained from the environmental air. The advantage of this embodiment is that the weight of about 4 kg water can be omitted for the portable unit because the hydrogene only needs to be provided. The portable unit can be bery light, especially because efficient burner are available. The heat resulting from the detonation gas reaction can be used for evaporating and concentrating the used dialysate after leaving the dialyzing unit, such that the portable unit will exhaust heated and moist air. The concentration should be adjusted such that the uremic toxine can still be rinsed with liquid into the basis unit when the interface is connected.

The advantage of having less weight of the portable unit has the disadvantage of the loss of water and energy into the environment. Therefore this embodiment is useful for regions where light portable units are needed and sufficient water and energy is available.

Another advantage of this embodiment ist hat therer is no need to transfer hygenic fresh water via the interface because the water is generated in the portable unit.

According to the invention all components may be provided in the portable unit including a reasonable amount of material. Then no separation into base unit and portable unit may be necessary. The ultrafiltrate can be fed directly to the electrolysis unit and the water generated in the fuel cell can be fed after adding the additives directly to the dialyzing unit. The energy of the fuel cell is used to operate the electrolysis unit. This embodiment may be portable especially considering future developments of the components needed.

According to the invention the basis unit and the portable unit can be connected via an interface. The interface may be designed such that a quick computer controlled transfer of the materials can be obtained, i.e. decharging used dialysate, recharging fresh dialysate, electrolyte, additives and hydrogen and optional water. The hydrogen may be charged under pressure.

The interface can be designed such that the amounts of material needed for a few hours dialysis can be exchanged within a few minutes, e.g. 1 to 2 min. The use of hydrogen can avoid the charging time accumulators, which could not be charged in this time. The energy needed can be provided in short time to the portable unit, simliar to filling the tank of a car with gas..

The invention is described with reference to the figures showing embodiments of the invention, wherein the following reference signs will be used:
- 1: blood inlet
- 2: blood outlet
- 3: blood pump
- 4: dialyzer
- 5: ultrafiltrate transfer
- 6: dialysate inlet
- 7: dialysate outlet
- 8: SN or DN catheter
- 9: volume balancing unit
- 10: priming shunt
- 11: priming pump
- 12: ultrafiltrate dosage pump
- 13: spent dialysate reservoir
- 14: electrolysis unit
- 15: split gas exhaust means
- 16: garbage residuals line
- 17: heating means
- 18: hydrogen separation /pore size filter and/or molecular sieve
- 19: hydrogen pump
- 20: hydrogen reservoir
- 21: hydrogen fuel cell
- 22: pure water reservoir
- 23: photovoltaic solar panels
- 24: power and control unit
- 25: store means, e.g. NaCl, Bic, Anticoag
- 26: electrical power supply line
- 27: portable hydrogen reservoir
- 28: hydrogen fuelk cell for power supply
- 29: pure water reservoir
- 30: fresh dialysate pump
- 31: local storage means, e.g. NaCl, Bic, Anticoag
- 32: dosage/admixture unit
- 33: power and control unit
- 34: docking interface
- 35: line for water from other sources (optional)
- 36: line for hydrogen from filter 18
- 37: oxygen from environment air
- 38: air
- 39: line for removing O₂, NH₃, Cl₂
- 40: oxygen from environment air
- 41: dosage line
- 42: vein
- 43: dialysate reservoir
- 44: base station
- 45: portable unit

### Brief description of the figures

- Fig. 1: is a schematic diagram of a first embodiment of the invention.
- Fig. 2: is a schematic diagram of an alternative embodiment of the invention with a portable dialysis unit.

### Detailed description of the embodiments

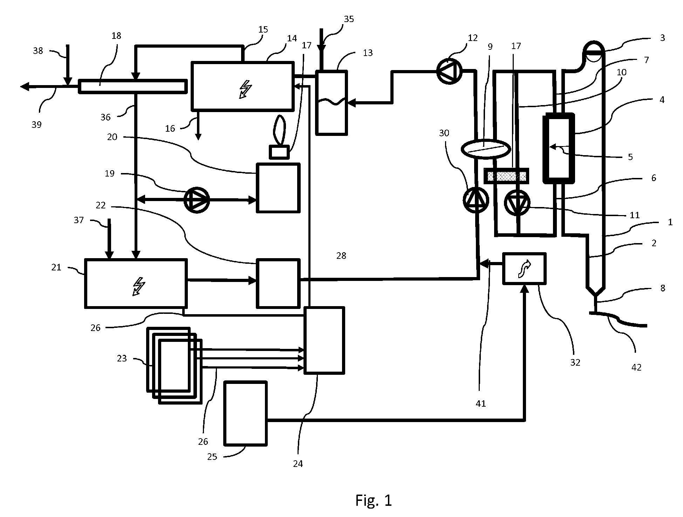

Fig.1 shows a first embodiment of the invention. A catheter 8 is inserted into a vein 42 of a patient. The catheter is y-shaped wherein one leg of the Y-catheter 8 is connected to the blood inlet 1 and the other leg of the y-catheter 8 blood outlet 2. The catheter 8 can be a single-needled (SN) or a double needed (DN) catheter. A blood pump 3 is provided to pump the blood of the patient from vein 42 to the dialyzer 4. In the dialyzer 4 an ultrafiltrate transfer 5 takes place. There is a dialysate circuit flowing in opposite direction through the dialyzer 4 having a dialysate inlet 6 and a dialysate outlet 7. In the dialysate circuit there is a priming shunt 10 and a priming pump 11 provided. Furthermore there is a volume balancing unit 9 coupling the dialysate circuit with a fresh water circuit. The specific design of the dialyzer and the above mentioned components are known to the skilled person.

An ultrafiltrate dosage pump 12 is pumping the dialysate to the spent dialysate reservoir 13. Optionally water from other sources can also feed into the spent dialysate reservoir 13 via optional line 35.

The dialysate is then fed into electrolysis unit 14. The electrolysis unit 14 can be a steam electrolysis unit or a cold electrolysis unit. Preferably a steam electrolysis unit is used because of the above mentioned advantages of lower energy consumption and less danger of clogging the anodes of the electrolysis unit. Preferably electrolysis unit 14 is a solid electrolyte cell operating with solid oxide electrochemical conversion.

The electrolysis unit 14 can be operated with alternating polarities of the electrical power. For instance AC power can be used. Alternatively pulsed DC power optionally with alternating polarities may be used. Using alternating polarities at appropriate frequencies will minimize the migration of the ions such that the gas separating element of the electrolysis unit may be omitted, especially in view of the separation of hydrogen in the hydrogen filter unit 18, which will be discussed in the following.

The gaseous components 02, H2, NH3 and C12 are fed through a split gas exhaust means 15 to a hydrogen separation filter 18. Garbage residuals like caustic lye, NaOH, NaCl, NH4Cl and NaOCl are removed for optional reuse via garbage residuals line 16.

There are several heating means 17 used to keep the materials at an appropriate temperature level. Heating means are known to the skilled person and do not need to described in more detail here.

The hydrogen separation filter 18 is preferably a pore size filter and/or a molecular sieve. In the embodiment shown the hydrogen separation filter 18 may be a microporeaus ceramic compound having a molecular diameter of about 100 to 230 pm (preferably 150 to 220 pm) such that hydrogen only can pass the filter and all other components are blocked.

The optimum working temperature of the hydrogen sepration filter 18 is higher than 130°C, preferably higher than 150°C, more preferred higher than 180°C. The optimum working temperature of the hydrogen sepration filter is less than 480°C, preferably less than 350°C, more preferred less than 250°C. The optimum working temperature of the hydrogen sepration filter 18 is most preferred at about 200°C.

The optimum working temperature of the electrolysis unit 14 is at about the same temperature as the optimum working temperature of the hydrogen sepration filter 18.

The filtered hydrogen is fed via line 36 to a hydrogen fuel cell 21 which is using environmental oxygen 37 to regain water. A hydrogen pump 19 is provided to fill excess hydrogen into a hydrogen reservoir 20 and to provide hydrogen therefrom if needed.

Other gaseous components like O₂, NH₃, Cl₂ are removed via line 39 and diluted with rinsing air 38.

The pure water generated in hydrogen fuel cell 21 is fed into pure water reservoir 22. There is a dosage/admixture unit 32 providing appropriate amounts from a storage means 25 to the pure water via dosage line 41, such as NaCl, Bic and anticoag, such that fresh dialysate is obtained for further usage. Pump 30 is driving this fresh dialysate to volume balancing unit 9.

Energy is provided by photovoltaic solar panels 23. Other power supply means can be used in addition and/or alternatively. Electrical power supply lines are provided for supplying electrical power to the various components. Some of them are shown in the figures. In addition there is a power and control unit 24 for driving the apparatus shown in the figures.

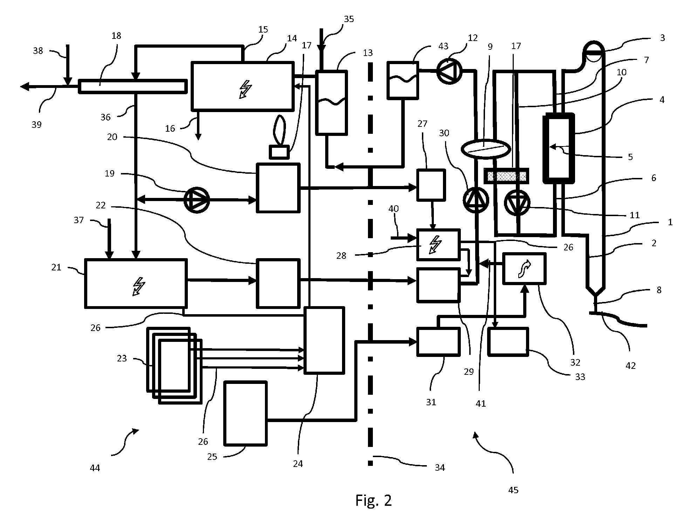

Fig. 2 shows another embodiment of the invention. The same reference signs are used to refer to same or similar components. Therefore reference is made to the above description of the embodiment shown in Fig. 1 and the differences only are discussed in the following.

The apparatus shown in Fig. 2 is separated into a base station 44 at the left hand side and a wearable or portable unit 45 at the right hand side. The wearable or portable unit is designed such that the patient can keep the portable unit at the person in order to have the option of continuous dialysis similar to the function of kidneys. The portable unit can be recharged in intervals at the base station. It will be possible to have one or more portable portions for one base station, depending on the size and capacity of the base station. It will also be possible to recharge and service the portable portion at different base stations.

The embodiment shown in Fig. 2 has a docking interface 34. As shown in Fig. 2 all reservoirs of Fig. 1 are separated into a large reservoir for the base station and a smaller reservoir for the portable unit.

Accordingly the portable unit 45 comprises a small dialysate reservoir 43 to be connected with the dialysate reservoir 13 of the base station 44, a small hydrogen reservoir 27 to be connected with the dialysate reservoir 20 of the base station 44, a small water reservoir 29 to be connected with the water reservoir 22 of the base station 44, and a small store means 31 to be connected with the store means 25 of the base station 44. The docking interface is designed such that the materials can be discharged (44 to 13) and recharged (20 to 27, 26 to 29 and 25 to 31) in a few minutes. Optionally energy can be charged into the portable unit if needed.

In addition the portable unit 45 comprises a local hydrogen fuel cell 28 to be used as power supply for the portable unit 45. To save weight, the local hydrogen fuel cell 28 uses oxygen 39 from the environmental air. Optional additional or alternative power supplies known to the skilled person can be provided.

The portable unit 45 comprises a local power and control unit 33.

Of course, the invention is not limited to the embodiments shown in the drawings. Therefore, the above description should not be considered limiting but explanatory. The below claims should be comprehended in such a way that a feature mentioned is present in at least one embodiment of the invention. This does not rule out the presence of further features. In so far as the claims and the above description define "first" and "second" features, this designation serves for distinguishing two similar features without determining an order.

## Claims

1. Apparatus for generating dialysate for dialysis comprising
an electrolysis unit (14) for electrolysing waste dialysate,
a hydrogen separation filter (18) for separating hydrogen from the electrolysed dialysate, and
a hydrogen fuel cell (21) for genarating pure water with the hydrogen obtained from the hydrogen separation filter and oxygen from the enviroment.

2. Apparatus according to any of the preceding claims, wherein the optimum working temperature of the hydrogen sepration filter is higher than 130°C, preferably higher than 150°C, more preferred higher than 180°C and most preferred at about 200°C.

3. Apparatus according to any of the preceding claims, wherein the optimum working temperature of the hydrogen sepration filter is less than 480°C, preferably less than 350°C, more preferred less than 250°C and most preferred at about 200°C.

4. Apparatus according to any of the preceding claims, wherein the optimum working temperature of the electrolysis unit is at about the same temperature as the optimum working temperature of the hydrogen sepration filter.

5. Apparatus according to any of the preceding claims, wherein the electrolysis unit (14) is a steam electrolysis unit.

6. Apparatus according to any of the preceding claims, wherein the electrolysis unit (14) is a solid electrolyte cell operating with solid oxide electrochemical conversion.

7. Apparatus according to any of the preceding claims, wherein the electrolysis unit (14) is operated with alternating polarities of the electrical power.

8. Apparatus according to any of claims 1 to 3, wherein the electrolysis unit (14) is a low temperature electrolysis unit.

9. Apparatus according to any of the preceding claims, wherein the hydrogen separation filter (18) is a pore size filter and/or a molecular sieve.

10. Apparatus according to any of the preceding claims, wherein the hydrogen separation filter (18) is a microporeaus ceramic compound.

11. Apparatus according to any of the preceding claims, being separated into a base station (44) and a portable unit (45).

12. Apparatus according to claim 11, comprising a docking interface (43) for connecting the base station (44) with the portable unit (45).

13. Apparatus according to claim 11, comprising a pluratliy of docking interfaces (43) for connecting a pluratilty of portable units (45) with the base station (44).

14. Apparatus according to claim 12 or 13, wherein the docking interface (43) comprises interfaces for connecting reservoirs provided in the base station (44) and the portable unit (45).

15. Method for generating dialysate for dialysis using an apparatus in accordance with any of the preceding claims.
